# EUROPEAN PATENT APPLICATION

(11) **EP 4 800 096 A1**
(43) Date of publication of application: **02.09.2026**
(21) Application number: 24897264.8
(22) Date of filing: 11.11.2024
(51) Int. Cl.: C12M 3/00

(54) **CULTURE DEVICE AND CULTURE SYSTEM**

(30) Priority: 30.11.2023 JP 2023203197
(71) Applicant: PHC HOLDINGS CORPORATION, Tokyo 100-8403 (JP)
(72) Inventor: TSUKAMOTO, Chihiro, Toon-shi, Ehime 791-0395 (JP); YOSHIDA, Kaori, Toon-shi, Ehime 791-0395 (JP); YONEHARA, Ryo, Toon-shi, Ehime 791-0395 (JP)
(74) Representative: Grünecker Patent- und Rechtsanwälte PartG mbB
(86) International application number: PCT/JP2024/039975
(87) International publication number: WO 2025/115577

(57) **Abstract**

A culture apparatus is disposed in a culture environment generation apparatus during cell culture, and includes: a frame that is open forward and includes a left frame and a right frame facing each other in a left-right direction; and a rocker that is provided at the frame and rocks a culture container; wherein, the frame has a container disposition region in which the culture container, a supply container, and a discharge container are disposed between the left frame and the right frame.

## Description

### Technical Field

The present invention relates to a culture apparatus and a culture system.

### Background Art

In the related art, there is known a culture apparatus that cultures a culture product such as a cell or a microorganism in a culture chamber (see Patent Literature (hereinafter, referred to as PTL) 1). In such a culture apparatus, a culture container is disposed in a culture chamber during cell culture. The culture container contains cells to be cultured and a culture medium.

In addition, the above-described culture apparatus includes a pump that supplies the culture medium to the culture container. The pump supplies the culture medium to the culture container from the outside of the culture apparatus.

### Citation List

### Patent Literature

PTL 1
Japanese Unexamined Patent Application Publication (Translation of PCT Application) No. 2018-525009

### Summary of Invention

### Technical Problem

In the technical field of cell culture as described above, a culture apparatus and a culture system having a novel configuration are desired.

An object of the present invention is to provide a culture apparatus and a culture system each having a novel configuration.

### Solution to Problem

An aspect of a culture apparatus according to the present invention is a culture apparatus disposed in a culture environment generation apparatus during cell culture, and the culture apparatus includes: a frame that is open forward and includes a left frame and a right frame facing each other in a left-right direction; and a rocker that is provided at the frame and rocks a culture container; wherein, the frame has a container disposition region in which the culture container, a supply container, and a discharge container are disposed between the left frame and the right frame.

An aspect of a culture system according to the present invention includes: the culture apparatus described above; and the culture environment generation apparatus that has a culture space accommodating the culture apparatus.

### Advantageous Effects of Invention

According to the present invention, it is possible to provide a culture apparatus and a culture system each having a novel configuration.

### Brief Description of Drawings

FIG. 1 is a perspective view of a culture system according to an embodiment of the present invention;
FIG. 2 is a front view of the culture system;
FIG. 3 is a block diagram illustrating a configuration of the culture system;
FIG. 4 is a cross-sectional view of a culture environment generation apparatus;
FIG. 5 is a front view of a container unit;
FIG. 6 is a front view of a frame;
FIG. 7 is a front view of the culture apparatus;
FIG. 8 is a rear view of the culture apparatus;
FIG. 9 is a left-side view of the culture apparatus;
FIG. 10 is a right-side view of the culture apparatus;
FIG. 11 is a plan view of the culture apparatus;
FIG. 12 is a bottom view of the culture apparatus;
FIG. 13 is a front view of the culture apparatus in a state in which the culture container is rocked; and
FIG. 14 is a front view of the culture apparatus in a state in which the culture container is rocked.

### Description of Embodiments

Hereinafter, embodiments of the present invention will be described in detail with reference to the drawings. The embodiments described below are examples, and the present invention is not limited thereto.

### [Embodiment]

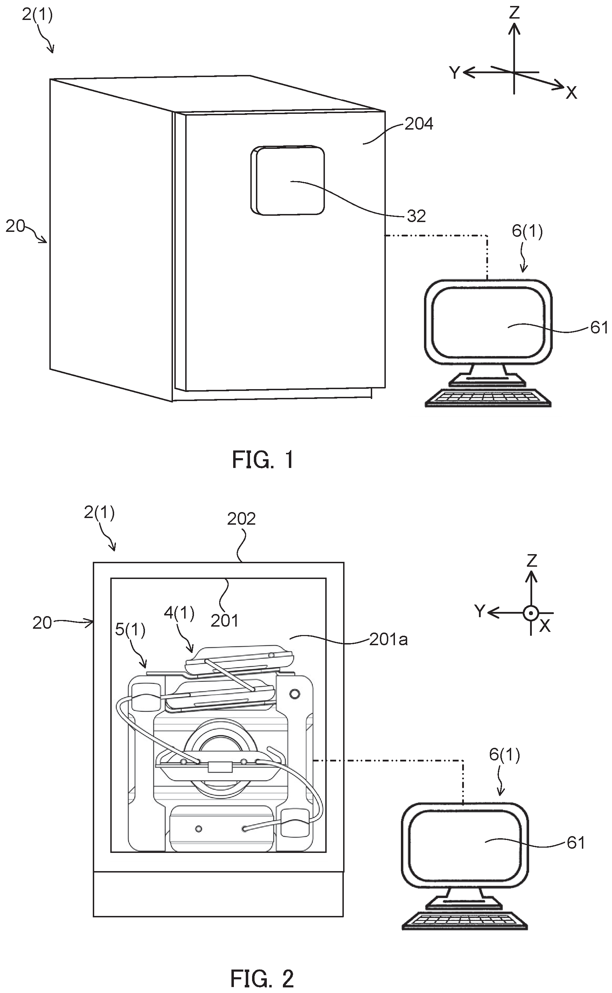

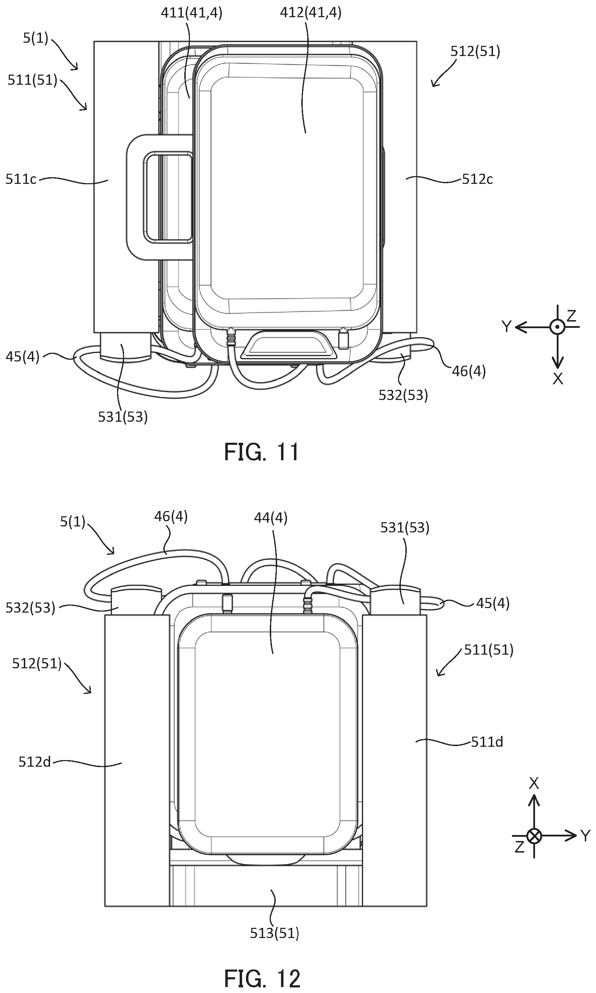

A configuration of culture system 1 according to an embodiment of the present invention will be described with reference to FIGS. 1 to 14.

In the following description, in describing the structure of each member constituting culture system 1, an orthogonal coordinate system (X, Y, Z) illustrated in each drawing may be used. The X direction coincides with a front-rear direction of culture environment generation apparatus 2 and culture apparatus 5 constituting culture system 1.

The X direction + side coincides with a front side of culture environment generation apparatus 2 and culture apparatus 5. The front side of culture environment generation apparatus 2 and culture apparatus 5 is a side on which a user faces when in use. The X direction - side coincides with a rear side of culture environment generation apparatus 2 and culture apparatus 5.

The Y direction coincides with a left-right direction of culture environment generation apparatus 2 and culture apparatus 5. The Y direction + side coincides with a left side when culture environment generation apparatus 2 and culture apparatus 5 are viewed from the front of culture environment generation apparatus 2 and culture apparatus 5. The Y direction - side coincides with a right side when culture environment generation apparatus 2 and culture apparatus 5 are viewed from the front of culture environment generation apparatus 2 and culture apparatus 5.

The Z direction coincides with an up-down direction of culture environment generation apparatus 2 and culture apparatus 5. The Z direction + side coincides with an upper side of culture environment generation apparatus 2 and culture apparatus 5. The upper side of culture environment generation apparatus 2 and culture apparatus 5 is a side away from a ground contact surface on which culture environment generation apparatus 2 and culture apparatus 5 are installed. The Z direction - side coincides with a lower side of culture environment generation apparatus 2 and culture apparatus 5.

As illustrated in FIGS. 1 to 3, culture system 1 includes culture environment generation apparatus 2, container unit 4, culture apparatus 5, and external terminal 6.

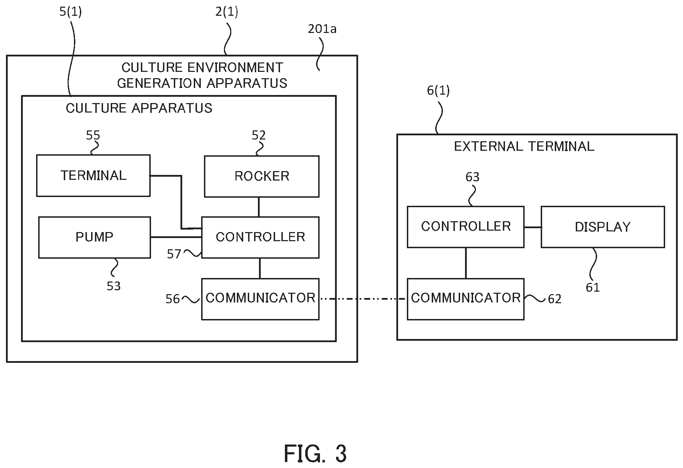

First, an outline of culture system 1 will be described. FIG. 1 is a perspective view of culture system 1 according to the embodiment. FIG. 2 is a front view of culture system 1 in which a part of the configuration is not illustrated. Further, FIG. 3 is a block diagram illustrating a configuration of culture system 1.

Culture environment generation apparatus 2 has a function of adjusting the environment of culture chamber 201a to an environment suitable for cell culture. Culture chamber 201a corresponds to an example of a culture space.

Culture apparatus 5 is disposed in culture chamber 201a of culture environment generation apparatus 2 while supporting container unit 4 during cell culture.

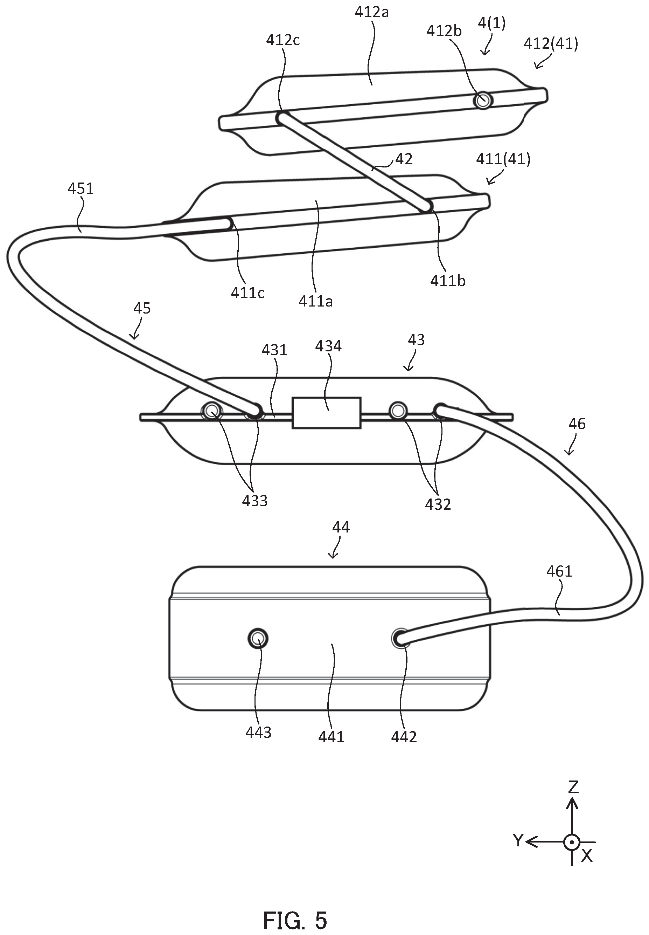

Then, cell culture is performed. Culture apparatus 5 has a function of rocking culture container 43 (see FIG. 5) of container unit 4 during cell culture.

Further, culture apparatus 5 has a function of acquiring information on a progress status of the cell culture from the culture medium contained in culture container 43 during cell culture.

Further, culture apparatus 5 has a function of transmitting the information on the progress status of the cell culture acquired from the culture medium contained in culture container 43 to external terminal 6. External terminal 6 displays the information on the progress status of the cell culture received from culture apparatus 5 on display 61.

As a result, the user can know the culture status of the target cells in real time based on the information on the culture medium displayed on display 61 of external terminal 6. Hereinafter, a specific configuration of culture system 1 will be described.

Culture environment generation apparatus 2 is an apparatus for adjusting the environment of culture chamber 201a formed inside a substantially box-shaped housing 20 to an environment suitable for cell culture, and an existing culture environment generation apparatus (for example, a CO₂ incubator) that is already on the market and is used in a laboratory or the like is used here. In such culture chamber 201a, cell culture or microorganism culture is performed. Hereinafter, the culture of cells, tissues, microorganisms, or the like is collectively referred to as cell culture. Cells, microorganisms, or the like that are targets of cell culture are collectively referred to as target cells.

Culture environment generation apparatus 2 has a function of adjusting the temperature, humidity, oxygen (O₂) concentration, and carbon dioxide (CO₂) concentration such that the atmosphere of culture chamber 201a is an environment (in other words, an atmosphere) suitable for culturing the target cells.

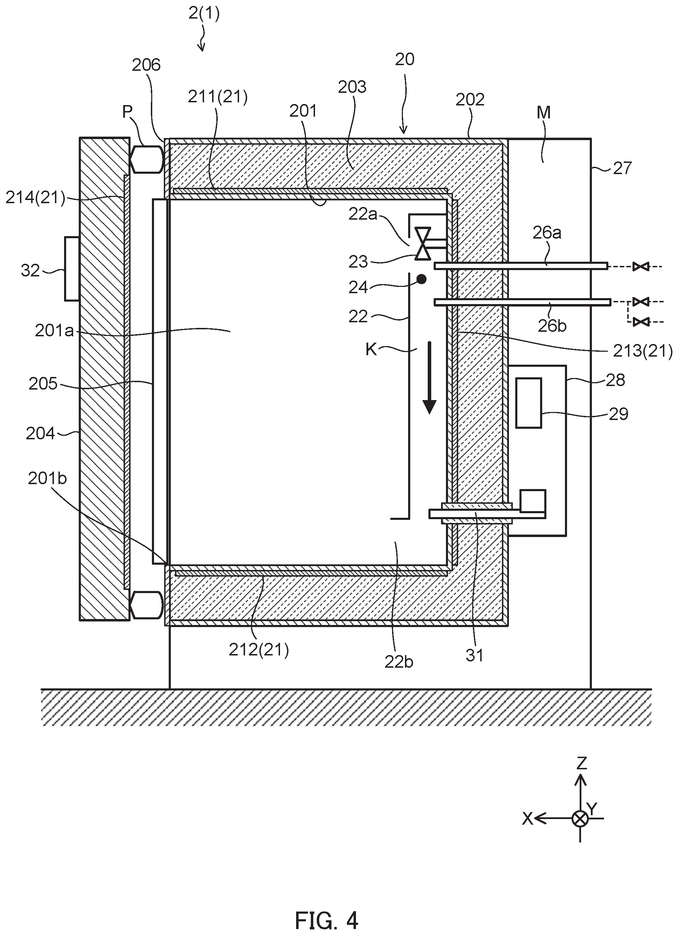

As illustrated in FIG. 4, housing 20 includes inner box 201, outer box 202, insulation 203, outer door 204, inner door 205, and front plate 206. FIG. 4 is a cross-sectional view of culture environment generation apparatus 2 when it is cut along the XZ plane and viewed from the Y direction - side (that is, the right side). In FIG. 4, culture apparatus 5 is not illustrated.

Inner box 201 has a substantially box shape made of metal. Inner box 201 includes culture chamber 201a on the inner side. In addition, inner box 201 has opening 201b of culture chamber 201a on the front surface. Culture chamber 201a has a size capable of accommodating culture apparatus 5. Culture chamber 201a accommodates culture apparatus 5 during cell culture.

Outer box 202 has a substantially box shape made of metal. Outer box 202 covers the outer surface of inner box 201 except for opening 201b (that is, a portion other than the front surface of inner box 201).

Insulation 203 is provided between inner box 201 and outer box 202. Insulation 203 is composed of a plurality of plate-shaped insulating members adhered to each other by an adhesive.

Outer door 204 is fixed to the front surface of outer box 202. Outer door 204 is pivotable. Inner door 205 is fixed to the front surface of inner box 201. Inner door 205 is pivotable. Such outer door 204 and inner door 205 open and close opening 201b.

Front plate 206 is a rectangular frame-shaped plate-like member. Front plate 206 is disposed along opening 201b. Front plate 206 connects a front end of inner box 201 and a front end of outer box 202 at a peripheral edge of opening 201b. Packing P is disposed between front plate 206 and the outer edge of outer door 204.

Further, culture environment generation apparatus 2 includes heating 21 that heats culture chamber 201a. Heating 21 includes a plurality of heaters. Each of the plurality of heaters has a plate shape. Specifically, heating 21 includes heaters 211, 212, 213, and 214, and a side surface heater (not illustrated).

Heaters 211, 212, 213, and 214 and the side surface heater are controlled by control apparatus 29, which will be described later, such that a temperature distribution in culture chamber 201a is uniform.

Culture environment generation apparatus 2 includes duct 22 in culture chamber 201a. Duct 22 includes gas passage K therein.

Culture environment generation apparatus 2 includes circulation blower 23 in gas passage K. Circulation blower 23 sucks air from culture chamber 201a at suction port 22a formed in an upper portion of duct 22.

Circulation blower 23 then sends the air sucked into duct 22 to the lower side of duct 22. The air sent to the lower side of duct 22 by circulation blower 23 is blown out to culture chamber 201a from discharge port 22b provided in a lower portion of duct 22. As a result, the air circulates in culture chamber 201a.

Culture environment generation apparatus 2 includes temperature sensor 24 in duct 22. Temperature sensor 24 detects the temperature of culture chamber 201a. Culture environment generation apparatus 2 includes a humidity sensor (not illustrated). The humidity sensor detects the humidity of culture chamber 201a.

Culture environment generation apparatus 2 includes gas supply apparatuses 26a and 26b in duct 22. Gas supply apparatuses 26a and 26b supply an adjustment gas to culture chamber 201a to adjust the O₂ gas concentration and the CO₂ gas concentration of culture chamber 201a. The adjustment gas may include, for example, an O₂ gas, a nitrogen (N₂) gas, and a CO₂ gas.

Culture environment generation apparatus 2 includes cover 27 that covers a rear surface and a bottom surface of outer box 202. A space between the rear surface of outer box 202 and cover 27 is machine room M for disposing various devices.

Culture environment generation apparatus 2 includes electric box 28 in machine room M. Culture environment generation apparatus 2 includes control apparatus 29 in electric box 28.

Culture environment generation apparatus 2 may include a steam supply apparatus that supplies steam to culture chamber 201a.

Culture environment generation apparatus 2 includes dehumidifying apparatus 31. Dehumidifying apparatus 31 includes a dehumidifying member that is a metal rod. Dehumidifying apparatus 31 prevents condensation in culture chamber 201a by dehumidifying culture chamber 201a. That is, dehumidifying apparatus 31 functions as a humidity controller.

Culture environment generation apparatus 2 having the above-described configuration receives instructions to start and stop culture environment generation apparatus 2, settings of the operating mode, and inputs of various set values of culture chamber 201a from operation panel 32 provided on outer door 204.

Then, control apparatus 29 controls each element constituting culture environment generation apparatus 2 based on the information input via operation panel 32 (in other words, the control panel). As a result, the environment of culture chamber 201a becomes an atmosphere suitable for cell culture.

Next, the configuration of container unit 4 will be described. FIG. 5 is a front view of container unit 4. Container unit 4 includes supply container 41, culture container 43, discharge container 44, supply tube 45, and discharge tube 46.

Supply container 41 contains a culture medium for supply (in other words, an unused culture medium). The culture medium for supply is supplied to culture container 43 by supply pump 531 described later.

In the case of the present embodiment, supply container 41 includes lower supply container 411, upper supply container 412, and connection tube 42. Lower supply container 411 and upper supply container 412 each contain a culture medium for supply. In the case of the present embodiment, supply container 41 is a two-stage supply container stacked in upper and lower stages.

Lower supply container 411 includes container body 411a, first port 411b, and second port 411c.

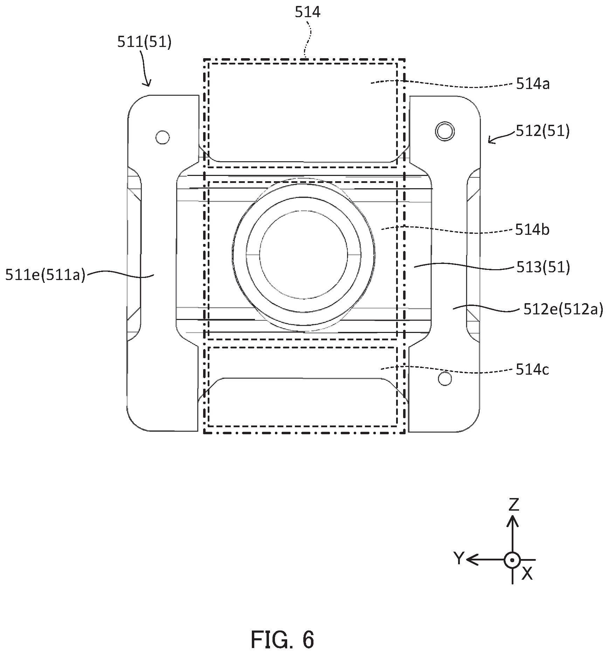

Container body 411a is a bag-shaped container having flexibility. First port 411b and second port 411c are each provided at the front end of container body 411a. Lower supply container 411 is disposed in supply container disposition region 514a (see FIG. 6) of culture apparatus 5 during cell culture in a state in which first port 411b and second port 411c are disposed in front.

First port 411b is provided in a half portion on one side (in the case of the present embodiment, the right half portion) at the front end of container body 411a. Further, second port 411c is provided in the other half portion (in the case of the present embodiment, the left half portion) at the front end of container body 411a.

First port 411b is a port through which the culture medium supplied from upper supply container 412 flows into lower supply container 411.

Second port 411c is a port through which the culture medium flows out from lower supply container 411 toward culture container 43. Second port 411c corresponds to an example of an outflow port of the supply container.

Lower supply container 411 is disposed in a state of being inclined such that the right end portion is higher than the left end portion during cell culture. In other words, lower supply container 411 is inclined such that the side of second port 411c, which is the outflow port, is lower during cell culture.

Accordingly, the culture medium contained in lower supply container 411 is collected at the left end portion due to gravity. That is, the culture medium contained in lower supply container 411 is collected on the second port 411c side. As a result, even in a case where there is a small amount of culture medium in lower supply container 411, the culture medium is likely to flow out from second port 411c.

Upper supply container 412 has the same configuration as lower supply container 411. Specifically, upper supply container 412 includes container body 412a, first port 412b, and second port 412c.

Container body 412a is a bag-shaped container having flexibility. First port 412b and second port 412c are each provided at the front end of container body 412a. Upper supply container 412 is supported by culture apparatus 5 in a state where first port 412b and second port 412c are disposed in front during cell culture.

First port 412b is provided in a half portion on one side (in the present embodiment, the right half portion) at the front end of container body 412a. In the present embodiment, first port 412b is closed.

In a case of a three-stage supply container in which a separate supply container (not illustrated) is disposed above upper supply container 412, first port 412b of upper supply container 412 is connected to the second port of the separate supply container by a connection tube (not illustrated). In this case, first port 412b of upper supply container 412 functions as a port through which the culture medium moving from the separate supply container flows into upper supply container 412.

Second port 412c is provided on the other side (in the present embodiment, the left half portion) at the front end of container body 412a. Second port 412c is a port through which the culture medium supplied from upper supply container 412 flows out to lower supply container 411.

Upper supply container 412 is disposed in a state of being inclined such that the right end portion is higher than the left end portion during cell culture. In other words, upper supply container 412 is inclined such that the side of second port 412c, which is the outflow port, is lower during cell culture.

Therefore, the culture medium contained in upper supply container 412 is collected at the left end portion due to gravity. That is, the culture medium contained in upper supply container 412 is collected on the second port 412c side. As a result, even in a case where there is a small amount of culture medium in upper supply container 412, the culture medium is likely to flow out from second port 412c.

Culture container 43 contains the culture medium and target cells. The target cells are cells that are the targets of cell culture. Such culture container 43 includes container body 431, first port 432, second port 433, and container-side sensor 434.

Before the cell culture is started in culture apparatus 5, the target cells and the culture medium are contained in culture container 43. When the cell culture is started in culture apparatus 5, the culture medium is supplied from supply container 41 to culture container 43.

Container body 431 is a bag-shaped container having flexibility. In addition, the container has a water vapor transmission rate and a gas transmission rate that are optimally set according to the cells and the culture medium to be contained. By using such a container, the environment in the container can be the same as the environment in culture environment generation apparatus 2. First port 432 and second port 433 are each provided at the front end of container body 431. Culture container 43 is disposed in culture container disposition region 514b (see FIG. 6) of culture apparatus 5 during cell culture in a state where first port 432 and second port 433 are disposed in front.

First port 432 is provided in a half portion on one side (in the case of the present embodiment, the right half portion) at the front end of container body 431.

In the case of the present embodiment, first port 432 is configured by a pair of ports provided side by side on the left and right. Only one port (in the case of the present embodiment, the right port) of the pair of ports constituting first port 432 is used. The other port (in the case of the present embodiment, the left port) of the pair of ports constituting first port 432 is closed.

Further, second port 433 is provided on the other side (in the case of the present embodiment, the left half portion) at the front end of container body 431.

In the case of the present embodiment, second port 433 is configured by a pair of ports provided side by side on the left and right. Only one port (in the case of the present embodiment, the right port) of the pair of ports constituting second port 433 is used. The other port (in the case of the present embodiment, the left port) of the pair of ports constituting second port 433 is closed.

First port 432 is a port through which the culture medium flows out from culture container 43 toward discharge container 44. First port 432 corresponds to an example of an outflow port of the culture container.

Second port 433 is a port through which the culture medium supplied from supply container 41 (specifically, lower supply container 411) flows into culture container 43. Second port 433 corresponds to an example of an inflow port of the culture container.

Container-side sensor 434 is provided at the central portion of the front end of container body 431. In other words, container-side sensor 434 is provided between first port 432 and second port 433 at the front end of container body 431.

A first end portion of container-side sensor 434 is exposed to the outside of culture container 43 (that is, container body 431). On the other hand, a second end portion of container-side sensor 434 is disposed inside culture container 43 (that is, container body 431). A reagent is provided at the second end portion of container-side sensor 434. The second end portion of container-side sensor 434 is in contact with the culture medium contained in culture container 43 (that is, container body 431). Such container-side sensor 434 is a sensor that detects a state of the culture medium based on an electrical signal generated by a reaction of the reagent provided at the second end portion.

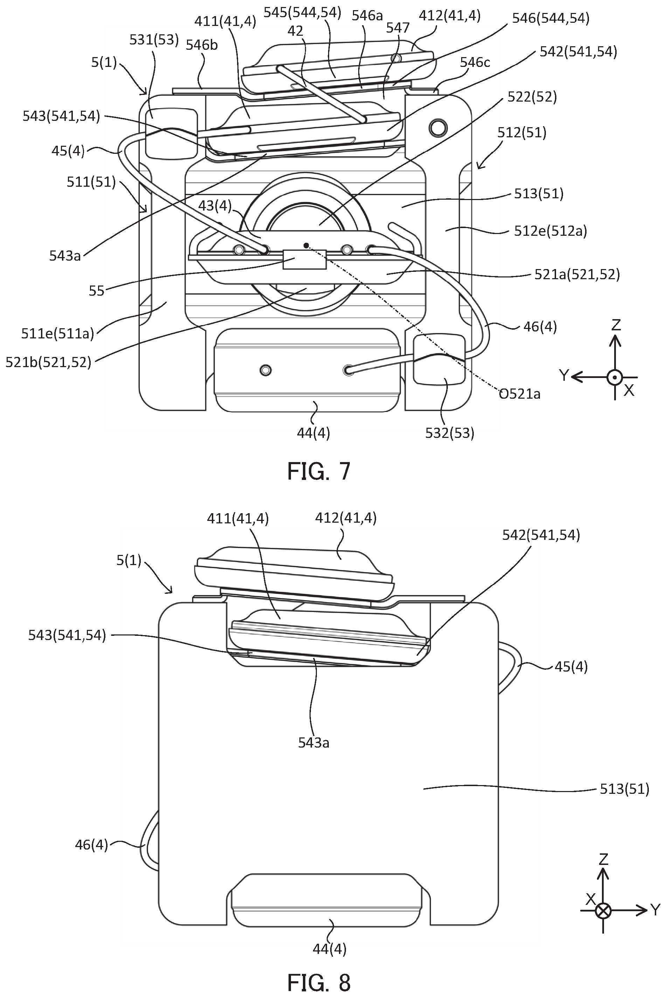

Discharge container 44 contains the culture medium discharged from culture container 43 (in other words, a used culture medium). Discharge container 44 is an empty container before cell culture. The culture medium contained in culture container 43 is discharged to discharge container 44 by discharge pump 532 (see FIG. 7), which is described later. The culture medium in discharge container 44 increases as the cell culture progresses.

Discharge container 44 includes container body 441, first port 442, and second port 443.

Container body 441 is a box-shaped container made of a resin. In the case of the present embodiment, container body 441 does not have flexibility. That is, container body 441 has rigidity enough to maintain its own shape.

First port 442 and second port 443 are each provided at a front end of container body 441. Discharge container 44 is disposed in discharge container disposition region 514c of culture apparatus 5 during cell culture in a state in which first port 442 and second port 443 are disposed in front.

First port 442 is provided in a half portion on one side (in the case of the present embodiment, the right half portion) at the front end of container body 441. First port 442 is a port into which the culture medium discharged from culture container 43 flows. First port 442 corresponds to an example of an inflow port of the discharge container.

Further, second port 443 is provided on the other side (in the case of the present embodiment, the left half portion) at the front end of container body 441. In the case of the present embodiment, second port 443 is not used. That is, second port 443 is closed.

Supply tube 45 connects second port 411c of supply container 41 (specifically, lower supply container 411) and second port 433 of culture container 43. Supply tube 45 has pump attachment 451 at the intermediate portion in the length direction. Pump attachment 451 is a portion that is attached to supply pump 531 during cell culture.

Discharge tube 46 connects first port 432 of culture container 43 and first port 442 of discharge container 44. Discharge tube 46 has pump attachment 461 at the intermediate portion in the length direction. Pump attachment 461 is a portion that is attached to discharge pump 532 during cell culture.

Container unit 4 having the above-described configuration is assembled into the state illustrated in FIG. 5 by the user outside culture environment generation apparatus 2 in the preparation work (in other words, the pre-treatment) for cell culture. Thus, the user can easily perform the assembly work of container unit 4.

Then, container unit 4 is mounted on culture apparatus 5 inside culture environment generation apparatus 2 (see FIG. 7). In the case of the present embodiment, culture apparatus 5 has a configuration in which container unit 4 is easily mounted from the front inside culture environment generation apparatus 2. This results in high efficiency of the work of mounting container unit 4 on culture apparatus 5. The configuration of culture apparatus 5 will be described later. Note that the shape of container unit 4 illustrated in FIG. 5 is the shape of container unit 4 in a state of being mounted on culture apparatus 5 (hereinafter, also referred to as a mounted state of container unit 4).

The state of container unit 4 and culture apparatus 5 accommodated in culture chamber 201a of culture environment generation apparatus 2 may be referred to as a use state of container unit 4 and culture apparatus 5.

In the use state of container unit 4, supply container 41 (specifically, upper supply container 412 and lower supply container 411) is supported by supply container support 54 (see FIG. 7) of culture apparatus 5.

In this state, ports 411b, 411c, 412b, and 412c of supply container 41 (specifically, lower supply container 411 and upper supply container 412) are disposed in front.

Therefore, the user can easily access each of ports 411b, 411c, 412b, and 412c of supply container 41 (specifically, lower supply container 411 and upper supply container 412) from the front of culture apparatus 5. This improves work efficiency of the replacement work or the like of supply container 41 (specifically, lower supply container 411 and upper supply container 412).

In addition, in the use state of container unit 4, culture container 43 is supported by rocker 52 (see FIG. 7) of culture apparatus 5. In this state, ports 432 and 433 of culture container 43 are disposed in front. Therefore, the user can easily access each of ports 432 and 433 of culture container 43 from the front of culture apparatus 5. This improves work efficiency of the replacement work or the like of culture container 43.

In addition, in the use state of container unit 4, discharge container 44 is placed at the bottom portion of culture chamber 201a (see FIG. 2). In this state, ports 442 and 443 of discharge container 44 are disposed in front. Therefore, the user can easily access each of ports 442 and 443 of discharge container 44 from the front of culture apparatus 5. This improves work efficiency of the replacement work or the like of discharge container 44.

In addition, in the use state of container unit 4, supply container 41 (specifically, lower supply container 411 and upper supply container 412), culture container 43, and discharge container 44 are connected by tubes (specifically, connection tube 42, supply tube 45, and discharge tube 46).

Further, in the use state of container unit 4, supply tube 45 and discharge tube 46 are routed in an S-shape in a front view (see FIG. 7) when viewed from the front of culture apparatus 5. As is clear from FIG. 7, in the case of the present embodiment, the routing of connection tube 42, supply tube 45, and discharge tube 46 is simple. Such a configuration contributes to the miniaturization of culture apparatus 5 and the improvement of the work efficiency of the assembly work of container unit 4. Further, the user can easily perform the work of mounting supply tube 45 and discharge tube 46 on supply pump 531 and discharge pump 532.

Next, the configuration of culture apparatus 5 will be described. As illustrated in FIG. 2, culture apparatus 5 is disposed in culture chamber 201a of culture environment generation apparatus 2 in a state in which container unit 4 is mounted during cell culture.

FIG. 2 is a front view of culture environment generation apparatus 2 in a state in which culture apparatus 5 is accommodated in culture chamber 201a. In FIG. 2, outer door 204 and inner door 205 of culture environment generation apparatus 2 are not illustrated.

Culture apparatus 5 is configured to be able to be taken in and out of culture chamber 201a of culture environment generation apparatus 2. In other words, culture apparatus 5 is configured as an apparatus separate from culture environment generation apparatus 2. Hereinafter, a specific configuration of culture apparatus 5 will be described.

Culture apparatus 5 includes frame 51, rocker 52, pump 53, supply container support 54, terminal 55, communicator 56, and controller 57.

Frame 51 is a member that supports rocker 52, pump 53, supply container support 54, terminal 55, communicator 56, and controller 57 that constitute culture apparatus 5. In other words, frame 51 is a member that unitizes rocker 52, pump 53, supply container support 54, terminal 55, communicator 56, and controller 57 that constitute culture apparatus 5.

Frame 51 is made of metal or synthetic resin. Frame 51 includes left frame 511, right frame 512, and rear frame 513.

Frame 51 has a hexahedral shape (specifically, a cubic shape or a rectangular parallelepiped shape). Left frame 511 is provided in a portion corresponding to a left side surface in a case where frame 51 is regarded as a hexahedron.

Right frame 512 is provided in a portion corresponding to a right side surface in a case where frame 51 is regarded as a hexahedron. That is, left frame 511 and right frame 512 face each other in the left-right direction. Rear frame 513 is provided in a portion corresponding to a rear side surface in a case where frame 51 is regarded as a hexahedron.

In frame 51, portions corresponding to a front side surface, an upper side surface, and a lower side surface in a case where frame 51 is regarded as a hexahedron are open. That is, frame 51 is open forward, upward, and downward.

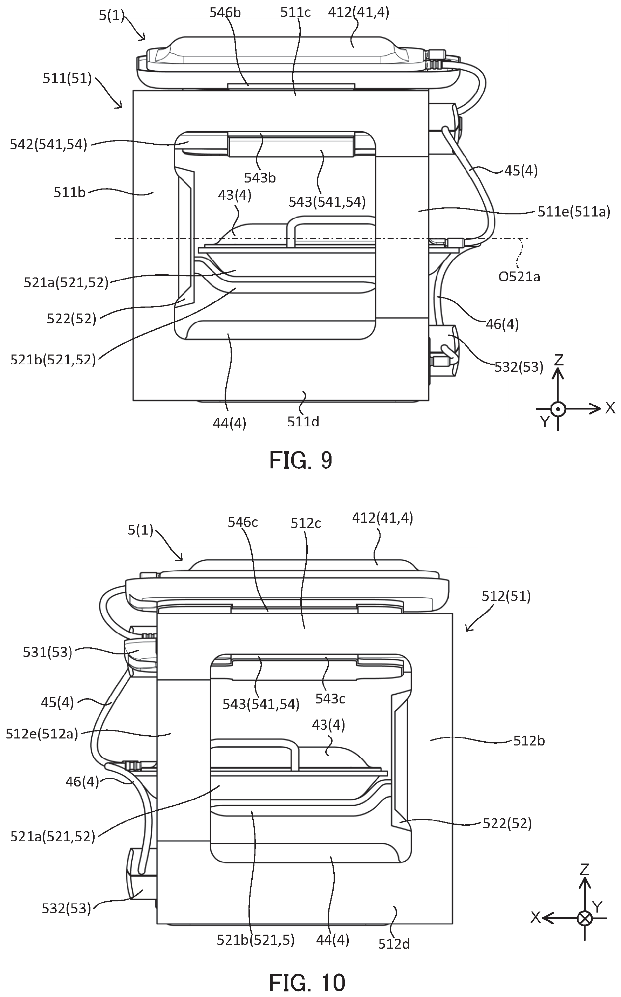

As illustrated in FIG. 9, left frame 511 has a rectangular frame shape parallel to the XZ plane. Therefore, the user can visually recognize rocker 52, which will be described later, and culture container 43 mounted on mount 521a of rocker 52 from the left side of left frame 511.

Specifically, as illustrated in FIG. 9, left frame 511 includes front edge 511a, rear edge 511b, upper edge 511c, and lower edge 511d.

Front edge 511a, rear edge 511b, upper edge 511c, and lower edge 511d are connected in a rectangular frame shape.

Front edge 511a has an axial shape extending in the up-down direction. Front edge 511a constitutes a front edge of left frame 511. Front edge 511a includes left handle 511e at an intermediate portion in the up-down direction.

In other words, left frame 511 includes left handle 511e at the front end of left frame 511. Left handle 511e is a portion that is gripped by the user when moving culture apparatus 5.

As illustrated in FIG. 7, left handle 511e has a smaller dimension (in other words, a width dimension) in the left-right direction than the upper end portion and the lower end portion of front edge 511a. Such a shape of left handle 511e is easy for the user to grip.

Rear edge 511b has an axial shape extending in the up-down direction. Rear edge 511b constitutes a rear edge of left frame 511. Front edge 511a and rear edge 511b face each other in the front-rear direction.

Upper edge 511c has an axial shape extending in the front-rear direction. Upper edge 511c constitutes an upper edge of left frame 511. Upper edge 511c connects the upper end portion of front edge 511a and the upper end portion of rear edge 511b in the front-rear direction.

Lower edge 511d has an axial shape extending in the front-rear direction. Lower edge 511d constitutes a lower edge of left frame 511. Lower edge 511d is a portion that is mounted on the bottom surface of culture chamber 201a during cell culture.

Lower edge 511d connects the lower end portion of front edge 511a and the lower end portion of rear edge 511b in the front-rear direction. Lower edge 511d and upper edge 511c face each other in the up-down direction.

As illustrated in FIG. 10, right frame 512 has a rectangular frame shape parallel to the XZ plane. Therefore, the user can visually recognize rocker 52, which will be described later, and culture container 43 mounted on mount 521a of rocker 52 from the right side of right frame 512.

Specifically, right frame 512 includes front edge 512a, rear edge 512b, upper edge 512c, and lower edge 512d.

Front edge 512a, rear edge 512b, upper edge 512c, and lower edge 512d are connected in a rectangular frame shape.

Front edge 512a has an axial shape extending in the up-down direction. Front edge 512a constitutes a front edge of right frame 512. Front edge 512a includes right handle 512e at an intermediate portion in the up-down direction.

In other words, right frame 512 includes right handle 512e at the front end of right frame 512. Right handle 512e is a portion that is gripped by the user when moving culture apparatus 5.

As illustrated in FIG. 7, right handle 512e has a smaller dimension (in other words, a width dimension) in the left-right direction than the upper end portion and the lower end portion of front edge 512a. Such a shape of right handle 512e is easy for the user to grip.

Rear edge 512b has an axial shape extending in the up-down direction. Rear edge 512b constitutes a rear edge of right frame 512. Front edge 512a and rear edge 512b face each other in the front-rear direction.

Upper edge 512c has an axial shape extending in the front-rear direction. Upper edge 512c constitutes an upper edge of right frame 512. Upper edge 512c connects the upper end portion of front edge 512a and the upper end portion of rear edge 512b in the front-rear direction.

Lower edge 512d has an axial shape extending in the front-rear direction. Lower edge 512d constitutes a lower edge of right frame 512. Lower edge 512d is a portion that is mounted on the bottom surface of culture chamber 201a during cell culture.

Lower edge 512d connects the lower end portion of front edge 512a and the lower end portion of rear edge 512b in the front-rear direction. Lower edge 512d and upper edge 512c face each other in the up-down direction.

Rear frame 513 has a rectangular plate shape parallel to the YZ plane. Rear frame 513 connects left frame 511 and right frame 512 in the left-right direction.

Rear edge 511b of left frame 511 is connected to the left end portion of rear frame 513. Left frame 511 is supported by rear frame 513 only at rear edge 511b. That is, left frame 511 is supported by rear frame 513 in a cantilevered manner.

Rear edge 512b of right frame 512 is connected to the right end portion of rear frame 513. Right frame 512 is supported by rear frame 513 only at rear edge 512b. That is, right frame 512 is supported by rear frame 513 in a cantilevered manner.

Frame 51 having the above-described configuration has container disposition region 514 (see FIG. 6) between left frame 511 and right frame 512 in which supply container 41, culture container 43, and discharge container 44 are disposed.

In FIG. 6, container disposition region 514 is indicated by a two-dot chain line. Container disposition region 514 includes supply container disposition region 514a in which supply container 41 (see FIG. 5) is disposed. Supply container disposition region 514a is a region present at the upper end portion of container disposition region 514 in the up-down direction.

Container disposition region 514 includes culture container disposition region 514b in which culture container 43 (see FIG. 5) is disposed. Culture container disposition region 514b is a region present at the central portion of container disposition region 514 in the up-down direction. In other words, culture container disposition region 514b is a region present below supply container disposition region 514a.

Container disposition region 514 includes discharge container disposition region 514c in which discharge container 44 (see FIG. 5) is disposed. Discharge container disposition region 514c is a region present at the lower end portion of container disposition region 514 in the up-down direction. In other words, discharge container disposition region 514c is a region present below culture container disposition region 514b.

As described above, supply container disposition region 514a, culture container disposition region 514b, and discharge container disposition region 514c are arranged in the up-down direction between left frame 511 and right frame 512.

In other words, container disposition region 514 includes supply container disposition region 514a, culture container disposition region 514b, and discharge container disposition region 514c in this order from the top. Therefore, during cell culture, supply container 41, culture container 43, and discharge container 44 are arranged in container disposition region 514 in this order from the top.

Rocker 52 rocks culture container 43 under the control of controller 57 during cell culture. Specifically, rocker 52 rocks culture container 43 around a rotation axis parallel to the front-rear direction between left frame 511 and right frame 512 during cell culture.

Rocker 52 is supported by rear frame 513. Specifically, rocker 52 includes culture container support 521 and drive 522.

Culture container support 521 includes mount 521a and rocking support 521b. Mount 521a has a tray shape made of metal or resin. During cell culture, culture container 43 is mounted on mount 521a.

Rocking support 521b is a plate-shaped member made of metal or resin extending in the front-rear direction. The front end of rocking support 521b is fixed to the lower surface of mount 521a. Accordingly, rocking support 521b supports mount 521a from below.

Rocking support 521b is rockably supported by drive 522. Specifically, the rear end of rocking support 521b is supported by drive 522. Rocking support 521b is rockable around central axis O₅₂₁ₐ (see FIGS. 7 and 9) parallel to the front-rear direction.

Drive 522 is supported by rear frame 513. Drive 522 supports rocking support 521b. Rocking support 521b is supported by drive 522 only at the rear end. That is, rocking support 521b is supported by rear frame 513 in a cantilevered manner via drive 522.

Drive 522 (see FIG. 7) rocks rocking support 521b under the control of controller 57. Such drive 522 includes an electric motor (not illustrated). When drive 522 is driven, rocking support 521b rocks around central axis O₅₂₁ₐ (see FIGS. 7 and 9) parallel to the front-rear direction. Then, mount 521a rocks around central axis O₅₂₁ₐ. As a result, culture container 43 mounted on mount 521a also rocks around central axis O₅₂₁ₐ.

Pump 53 is provided on frame 51. Pump 53 moves the culture medium between supply container 41, culture container 43, and discharge container 44 under the control of controller 57. Pump 53 corresponds to an example of a medium transfer.

Pump 53 includes supply pump 531 and discharge pump 532.

Supply pump 531 supplies the culture medium contained in supply container 41 to culture container 43. Supply pump 531 is provided on the front surface of frame 51.

Specifically, supply pump 531 is provided at an upper end on the front surface of one of left frame 511 and right frame 512 (in the case of the present embodiment, left frame 511). That is, supply pump 531 is supported by left frame 511.

Supply tube 45 of container unit 4 is attached to supply pump 531 during cell culture. Supply pump 531 moves the culture medium in supply tube 45 in a direction from supply container 41 toward culture container 43. The direction from supply container 41 toward culture container 43 may be referred to as a supply direction.

Discharge pump 532 discharges the culture medium contained in culture container 43 to discharge container 44. Discharge pump 532 is provided on the front surface of frame 51.

Specifically, discharge pump 532 is provided at a lower end on the front surface of the other of left frame 511 and right frame 512 (in the case of the present embodiment, right frame 512). That is, discharge pump 532 is supported by right frame 512.

Discharge tube 46 of container unit 4 is attached to discharge pump 532. Discharge pump 532 moves the culture medium in discharge tube 46 in a direction from culture container 43 toward discharge container 44. The direction from culture container 43 toward discharge container 44 may be referred to as a discharge direction.

The unused culture medium contained in supply container 41 (specifically, lower supply container 411) flows out from second port 411c (in other words, the outflow port) of lower supply container 411, and then moves to supply pump 531, second port 433 (in other words, the inflow port) of culture container 43, culture container 43, first port 432 (in other words, the outflow port) of culture container 43, discharge pump 532, first port 442 (in other words, the inflow port) of discharge container 44, and discharge container 44 in this order.

As described above, in the case of the present embodiment, supply pump 531 and discharge pump 532 are provided at diagonal positions on the front surface of frame 51. Further, supply pump 531 is provided above discharge pump 532. Such a configuration contributes to the improvement of the work efficiency of the work of attaching supply tube 45 to supply pump 531 and the work of attaching discharge tube 46 to discharge pump 532.

Supply container support 54 is a member for supporting supply container 41. Supply container support 54 is supported by frame 51. Supply container support 54 is provided in supply container disposition region 514a in container disposition region 514 (see FIG. 6).

Supply container support 54 includes lower support 541 and upper support 544.

Lower support 541 includes lower mount 542 and lower support member 543.

Lower mount 542 has a tray shape made of metal or resin. During cell culture, lower supply container 411 is mounted on lower mount 542. Lower support member 543 is a member for supporting lower mount 542 with respect to frame 51.

Lower support member 543 includes support element 543a (see FIG. 7), left side fixer 543b (see FIG. 9), and right-side fixer 543c (see FIG. 10).

Support element 543a has a plate shape made of metal or resin and is fixed to a lower surface of lower mount 542. That is, support element 543a supports lower mount 542 from below. Support element 543a is inclined such that the right-end portion is higher than the left-end portion. In other words, support element 543a is inclined to be higher from the left-end portion toward the right-end portion.

Accordingly, lower mount 542 is inclined such that the right-end portion is higher than the left-end portion. In other words, lower mount 542 is inclined to be higher from the left-end portion toward the right-end portion.

Left side fixer 543b (see FIG. 9) is a portion for fixing lower support member 543 to left frame 511 of frame 51. Left side fixer 543b is connected to the left-end portion of support element 543a. Left side fixer 543b is fixed to a lower surface of upper edge 511c of left frame 511 by a fastening means (for example, a screw or a bolt).

Right side fixer 543c (see FIG. 10) is a portion for fixing lower support member 543 to right frame 512 of frame 51. Right side fixer 543c is connected to the right-end portion of support element 543a. Right side fixer 543c is fixed to a lower surface of upper edge 512c of right frame 512 by a fastening means (for example, a screw or a bolt).

Lower support 541 having the above-described configuration supports lower supply container 411 during cell culture. In this state, lower supply container 411 is inclined such that the right-end portion is higher than the left-end portion.

Upper support 544 is provided above lower support 541 in supply container disposition region 514a of frame 51. Upper support 544 includes upper mount 545 (see FIG. 7) and upper support member 546 (see FIG. 7).

Upper mount 545 has a tray shape made of metal or resin. During cell culture, upper supply container 412 is mounted on upper mount 545. Upper support member 546 is a member for supporting upper mount 545 with respect to frame 51.

Upper mount 545 is provided above lower mount 542. In addition, upper mount 545 is provided in a state where a part of upper mount 545 is offset to the right side with respect to lower mount 542.

As illustrated in FIG. 7, upper support member 546 includes support element 546a, left side fixer 546b, and right side fixer 546c.

Support element 546a has a plate shape made of metal or resin and is fixed to a lower surface of upper mount 545. That is, support element 546a supports upper mount 545 from below. Support element 546a is inclined such that the right-end portion is higher than the left-end portion. In other words, support element 546a is inclined to be higher from the left-end portion toward the right-end portion.

Accordingly, upper mount 545 is inclined with respect to the horizontal plane (that is, the XY plane) such that the right-end portion is higher than the left-end portion. In other words, upper mount 545 is inclined to be higher from the left-end portion toward the right-end portion with respect to the horizontal plane.

Note that upper mount 545 and lower mount 542 are substantially parallel to each other. That is, the inclination angle of upper mount 545 with respect to the horizontal plane and the inclination angle of lower mount 542 with respect to the horizontal plane are equal to or substantially equal to each other.

Left side fixer 546b is a portion for fixing upper support member 546 to left frame 511 of frame 51. Left side fixer 546b is connected to the left-end portion of support element 546a. Left side fixer 546b is fixed to the upper surface of upper edge 511c (see FIG. 9) of left frame 511 by a fastening means (for example, a screw or a bolt).

Right side fixer 546c is a portion for fixing upper support member 546 to right frame 512 of frame 51. Right side fixer 546c is connected to the right-end portion of support element 546a. Right side fixer 546c is fixed to the upper surface of upper edge 512c (see FIG. 10) of right frame 512 by a fastening means (for example, a screw or a bolt).

Upper support 544 having the above-described configuration supports upper supply container 412 during cell culture. In this state, upper supply container 412 is inclined such that the right-end portion is higher than the left-end portion.

In addition, as described above, upper mount 545 is provided in a state of being offset to the right side with respect to lower mount 542. Accordingly, upper supply container 412 mounted on upper mount 545 is offset to the right side with respect to lower supply container 411 mounted on lower mount 542. Therefore, the user can visually recognize the remaining amount of the culture medium contained in lower supply container 411 from above.

Further, as illustrated in FIG. 7, gap 547 is provided in the up-down direction between the upper surface of lower supply container 411 and the lower surface of upper support 544. Therefore, the user can easily visually recognize the remaining amount of the culture medium contained in lower supply container 411.

Note that culture apparatus 5 may include a remaining amount detector (not illustrated) that detects information on the remaining amount of the culture medium contained in upper supply container 412 and lower supply container 411. Such a remaining amount detector may include, for example, a sensor that detects the weight of upper supply container 412 and a sensor that detects the weight of lower supply container 411.

Alternatively, the above-described remaining amount detector may include a sensor that detects the volume (weight) of the culture medium that has flowed out from lower supply container 411 and a sensor that detects the volume (weight) of the culture medium that has flowed out from upper supply container 412.

Culture apparatus 5 may notify the user of the information on the remaining amount of the culture medium detected by the remaining amount detector. Specifically, the information on the remaining amount of the culture medium may be transmitted to external terminal 6, which will be described later, by communicator 56, which will also be described later. External terminal 6 may display the information on the remaining amount of the culture medium acquired from culture apparatus 5 on display 61 (see FIG. 1).

The user can see the information on the remaining amount of the culture medium displayed on display 61 of external terminal 6 and know the remaining amount of the culture medium contained in lower supply container 411 and upper supply container 412 in real time.

Terminal 55 (see FIG. 3) is a terminal connected to container-side sensor 434 provided on culture container 43. That is, terminal 55 is a terminal that connects culture apparatus 5 and container-side sensor 434. Culture apparatus 5 acquires information on the culture medium contained in culture container 43 from container-side sensor 434 via terminal 55. The information on the culture medium is information corresponding to the progress status of the cell culture. That is, the progress status of the cell culture can be measured based on the information on the culture medium.

The information on the culture medium includes information on glucose contained in the culture medium and information on lactic acid. In addition, the information on the culture medium may include information on pH, information on dissolved oxygen concentration (DO), information on glutamine, information on glutamic acid, information on calcium, information on magnesium, information on NADP+/NADPH, and information on NAD+/NADH.

The unused culture medium supplied from supply container 41 to culture container 43 contains, as nutrients, for example, glucose, lactose, vitamins, serum, amino acids, and the like. The target cells, which are the targets of the cell culture, increase in culture container 43 while consuming the nutrients contained in the culture medium.

The consumption rate of the nutrients contained in the culture medium is correlated with the rate at which the target cells increase. Thus, the culture status of the target cells can be measured based on the concentration of the nutrients (specifically, glucose) contained in the culture medium.

In addition, the target cells consume the nutrients (specifically, glucose) contained in the culture medium and discharge lactic acid, which is a metabolite, when the target cells increase. Thus, the culture status of the target cells can be measured based on the concentration of lactic acid contained in the culture medium or the production rate of lactic acid.

In the case of the present embodiment, container-side sensor 434 detects information on glucose contained in the culture medium and information on lactic acid as information on the culture medium. Then, the information on the culture medium detected by container-side sensor 434 is transmitted to external terminal 6 by communicator 56. External terminal 6 displays the information on the culture medium acquired from culture apparatus 5 on display 61 (see FIG. 1). Note that the sensor that detects the information on the culture medium may be provided in culture apparatus 5.

Container-side sensor 434 continuously detects the information on the culture medium in real time. Note that the information on the culture medium transmitted to external terminal 6 may be information obtained by sampling the information on the culture medium detected by container-side sensor 434 at a predetermined sampling cycle.

As described above, culture apparatus 5 according to the present embodiment has an inline monitoring function by container-side sensor 434 incorporated in culture container 43. The user can know the culture status of the target cells in real time based on the information on the culture medium displayed on display 61 of external terminal 6.

As illustrated in FIG. 7, terminal 55 is provided at the front end of mount 521a of rocker 52. Terminal 55 has a terminal (not illustrated) that is connected to container-side sensor 434.

Terminal 55 is electrically connected to the first terminal of container-side sensor 434 (see FIG. 5) of culture container 43. Terminal 55 is connected to controller 57. Terminal 55 applies a voltage to container-side sensor 434 under the control of controller 57. Container-side sensor 434 detects the information on the culture medium based on the applied voltage.

Communicator 56 (see FIG. 3) is communicatively connected to external terminal 6. Communicator 56 and external terminal 6 are connected by a cable in a wired manner. Alternatively, communicator 56 and external terminal 6 may be, for example, wirelessly connected by various wireless communication methods such as wireless LAN (for example, WiFi (registered trademark)) or Bluetooth (registered trademark). The connection method between communicator 56 and external terminal 6 is not particularly limited.

Communicator 56 transmits information to external terminal 6. Further, communicator 56 receives information from external terminal 6. Communicator 56 may transmit the information received from external terminal 6 to controller 57, which will be described below.

Controller 57 (see FIG. 3) comprehensively controls the operation of culture apparatus 5. Controller 57 is supported by frame 51 (for example, rear frame 513).

Specifically, controller 57 controls the operation of rocker 52. Controller 57 controls, for example, rocker 52 (specifically, drive 522) to control the rocking speed of rocker 52 (specifically, culture container support 521) (that is, the rocking speed of culture container 43).

Controller 57 rocks culture container support 521 at a predetermined constant speed. Alternatively, controller 57 may rock culture container support 521 based on the detected value of container-side sensor 434 (that is, the information on the culture medium).

Controller 57 controls the operation of pump 53 (specifically, supply pump 531 and discharge pump 532).

Specifically, controller 57 controls the timing of driving and stopping pump 53 (specifically, supply pump 531 and discharge pump 532). Controller 57 may control the timing of driving and stopping pump 53 (specifically, supply pump 531 and discharge pump 532) based on the detected value of container-side sensor 434 (that is, the information on the culture medium).

Further, controller 57 controls the flow rate of the culture medium supplied from supply container 41 to culture container 43 by supply pump 531. Hereinafter, the flow rate of the culture medium supplied from supply container 41 to culture container 43 by supply pump 531 is referred to as a supply flow rate of supply pump 531.

The supply flow rate of supply pump 531 may be a constant flow rate set in advance. That is, controller 57 may drive supply pump 531 at the constant supply flow rate set in advance. Alternatively, controller 57 may variably control the supply flow rate of supply pump 531 based on the detected value of container-side sensor 434 (that is, the information on the culture medium).

Further, controller 57 controls the flow rate of the culture medium discharged from culture container 43 to discharge container 44 by discharge pump 532. Hereinafter, the flow rate of the culture medium discharged from culture container 43 to discharge container 44 by discharge pump 532 is referred to as a discharge flow rate.

The discharge flow rate of discharge pump 532 may be a constant flow rate set in advance. That is, controller 57 may drive discharge pump 532 at the constant discharge flow rate set in advance. Alternatively, controller 57 may variably control the discharge flow rate of discharge pump 532 based on the detected value of container-side sensor 434 (that is, the information on the culture medium).

Further, controller 57 controls the communication operation of communicator 56. Controller 57 controls the communication operation of communicator 56 to transmit the information on the culture medium detected by container-side sensor 434 to external terminal 6.

In a case where communicator 56 receives a control command for culture apparatus 5 from external terminal 6, controller 57 acquires the control command for culture apparatus 5 from communicator 56. Controller 57 may control the operation of each element constituting culture apparatus 5 based on the control command for culture apparatus 5.

The control command received from external terminal 6 includes, for example, a rocking control command and a medium transfer control command.

Controller 57 controls rocker 52 based on, for example, the rocking control command acquired from external terminal 6. The rocking control command includes a control command related to the rocking operation (for example, the rocking speed and/or the rocking angle) of rocker 52.

In addition, controller 57 controls pump 53 (specifically, supply pump 531 and/or discharge pump 532) based on, for example, the medium transfer control command acquired from external terminal 6. The medium transfer control command includes a control command related to the medium transfer operation (for example, the supply flow rate and/or the discharge flow rate) of pump 53 (specifically, supply pump 531 and/or discharge pump 532).

Note that, when the culture medium in culture container 43 is discharged to discharge container 44, controller 57 controls drive 522 of rocker 52 to incline culture container 43 such that second port 433 is downward. Second port 433 of culture container 43 is a port connected to supply container 41 (that is, a supply port).

As a result, the target cells in culture container 43 are collected on the second port 433 side of culture container 43. In this state, controller 57 drives discharge pump 532 to discharge the culture medium in culture container 43 to discharge container 44. In this case, since the target cells in culture container 43 are collected on the second port 433 side of culture container 43, it is possible to suppress the discharge of the target cells to discharge container 44.

External terminal 6 is provided outside culture environment generation apparatus 2. External terminal 6 is, for example, a computer. External terminal 6 may be a desktop computer (personal computer, workstation, or the like), a laptop computer (personal computer, workstation, or the like), a tablet terminal, a mobile device such as a smartphone, or the like.

Further, external terminal 6 may be provided in culture environment generation apparatus 2. In this case, external terminal 6 may be operation panel 32 (in other words, the control panel) provided on outer door 204 of culture environment generation apparatus 2.

Focusing on the hardware configuration, external terminal 6 includes a processor (CPU), an input apparatus, an output apparatus, a memory, a communication interface, a storage, and the like, which are provided in a general computer.

Focusing on the function, external terminal 6 includes display 61, communicator 62, and controller 63. Display 61 displays information. Display 61 may be a general display (for example, a liquid crystal display).

Communicator 62 is connected to communicator 56 of culture apparatus 5. Communicator 62 acquires information from culture apparatus 5. The information acquired by communicator 62 from culture apparatus 5 is, for example, information on the culture medium. The information on the culture medium is detected by container-side sensor 434 provided on culture container 43, as described above.

Controller 63 controls the operations of display 61 and communicator 62. Controller 63 displays the information on the culture medium acquired from culture apparatus 5 by communicator 62 on display 61.

The information on the culture medium is detected in real time by container-side sensor 434 of culture container 43. Thus, the user can know the culture status of the target cells contained in culture container 43 by viewing the information on the culture medium displayed on display 61.

External terminal 6 receives a command for controlling the operation of culture apparatus 5 (hereinafter, referred to as a control command for culture apparatus 5) from the user. The user inputs the control command for culture apparatus 5 to external terminal 6 via the input apparatus.

The control command for culture apparatus 5 may include the rocking control command for controlling rocker 52 and the medium transfer control command for controlling pump 53 (specifically, supply pump 531 and discharge pump 532).

Controller 63 controls communicator 62 to transmit the control command for culture apparatus 5 input by the user to culture apparatus 5. Controller 57 (see FIG. 3) of culture apparatus 5 controls each element (specifically, rocker 52 and pump 53) of culture apparatus 5 based on the control command for culture apparatus 5 received from external terminal 6. That is, the user can remotely operate culture apparatus 5 by using external terminal 6.

### (Benefits and Effects of Present Embodiment)

Hereinafter, the work performed by the user to perform cell culture, the operation of culture system 1, and the benefits and effects of culture system 1 according to the present embodiment will be briefly described.

First, the user accommodates culture apparatus 5 in the existing culture environment generation apparatus 2. Then, the user assembles container unit 4 (see FIG. 5) outside the existing culture environment generation apparatus 2. Next, the user attaches container unit 4 to culture apparatus 5 inside culture environment generation apparatus 2. Note that, either the work of accommodating culture apparatus 5 in culture environment generation apparatus 2 or the work of assembling container unit 4 outside culture environment generation apparatus 2 may be performed first.

In performing the above work, the user attaches supply tube 45 of container unit 4 to supply pump 531. Further, the user attaches discharge tube 46 of container unit 4 to discharge pump 532. In the case of the present embodiment, supply pump 531 and discharge pump 532 are provided on the front surface of frame 51. Therefore, it is easy to perform the work of attaching supply tube 45 to supply pump 531 and the work of attaching discharge tube 46 to discharge pump 532.

Next, the user operates operation panel 32 of culture environment generation apparatus 2 to drive culture environment generation apparatus 2. Note that the timing of starting culture environment generation apparatus 2 may be appropriately determined.

Next, the user drives culture apparatus 5. When culture apparatus 5 is driven, rocker 52 and pump 53 of culture apparatus 5 are driven. Rocker 52 rocks culture container 43 mounted on mount 521a.

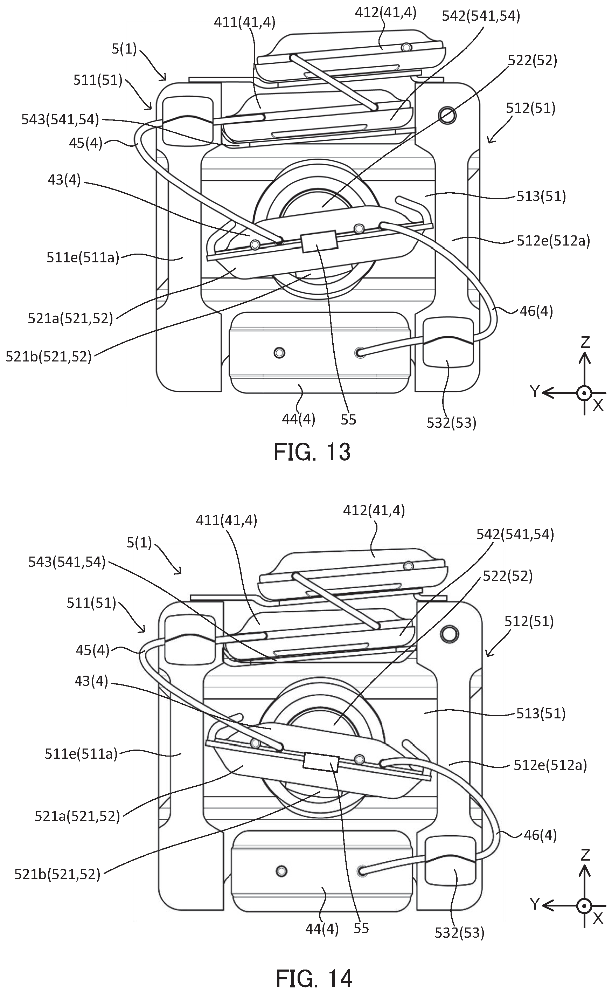

FIG. 13 is a diagram illustrating a state in which culture container 43 is rocked in the first direction from the reference position illustrated in FIG. 7. The first direction is a direction opposite to the clockwise direction in FIGS. 7 and 13. Further, FIG. 14 is a diagram illustrating a state in which culture container 43 is rocked in the second direction from the reference position illustrated in FIG. 7. The second direction is the clockwise direction in FIGS. 7 and 14.

Rocker 52 repeatedly rocks culture container 43 such that culture container 43 transitions between the state illustrated in FIG. 13 and the state illustrated in FIG. 14.

Further, pump 53 (specifically, supply pump 531) supplies the unused culture medium contained in supply container 41 (specifically, lower supply container 411) to culture container 43. Note that the timing at which the culture medium is supplied to culture container 43 by supply pump 531 and the flow rate of the culture medium supplied to culture container 43 by supply pump 531 may be controlled by controller 57.

Further, during cell culture, container-side sensor 434 provided on culture container 43 continuously detects information on the culture medium contained in culture container 43 in real time. The information on the culture medium detected by container-side sensor 434 is transmitted to external terminal 6 by communicator 56.

External terminal 6 displays the information on the culture medium acquired from culture apparatus 5 on display 61 (see FIG. 1). The user can know the culture status of the target cells in real time based on the information on the culture medium displayed on display 61 of external terminal 6.

Further, the user removes container unit 4 from culture chamber 201a after the cell culture is completed. Then, the user removes the cultured target cells from culture container 43. Further, the user disposes of the used culture container 43, supply container 41, and discharge container 44.

As described above, in the case of culture apparatus 5 according to the present embodiment, the user can perform the culture in an environment using the existing culture environment generation apparatus 2. Thus, existing equipment can be effectively utilized. Further, the user can perform the pre-treatment (in other words, the preparation work) of the cell culture and the post-treatment of the cell culture outside culture chamber 201a. Thus, the pre-treatment and the post-treatment are easily performed. As a result, the work efficiency of the work related to the cell culture is significantly improved.

Further, frame 51 of culture apparatus 5 according to the present embodiment has a shape that facilitates the cleaning work. This improves the work efficiency of the cleaning work of culture apparatus 5 in the above-described post-treatment.

Further, as is clear from FIG. 5, container unit 4 attached to culture apparatus 5 according to the present embodiment is simply configured. Thus, the user can easily assemble container unit 4.

Further, in the use state of container unit 4, supply container 41 (specifically, lower supply container 411 and upper supply container 412), culture container 43, and discharge container 44 are disposed such that each port is disposed in front. Thus, in the use state of container unit 4, the user can efficiently perform the replacement work and the like of supply container 41 (specifically, lower supply container 411 and upper supply container 412), culture container 43, and discharge container 44.

Further, in the use state of container unit 4, connection tube 42, supply tube 45, and discharge tube 46 are also disposed in front. Thus, in the use state of container unit 4, the user can efficiently perform the attachment/detachment work of connection tube 42, supply tube 45, and discharge tube 46.

In addition, the benefits and effects of culture apparatus 5 and culture system 1 according to the present embodiment are as described above.

### (Additional notes)

The disposition of each element constituting the culture apparatus is not limited to the disposition of each element constituting culture apparatus 5 according to the above-described embodiment. For example, the disposition of each element constituting the culture apparatus may be a disposition opposite to the disposition of each element constituting culture apparatus 5 illustrated in FIG. 7 in the left-right direction.

The example of the embodiment according to the present invention has been described above. The above description is an example of a suitable embodiment, and the technical scope of the present invention is not limited to the above-described embodiment. That is, the description of the configuration of the apparatus and the shape of each part described above is an example, and various modifications and additions to the configuration of the above-described embodiment are possible within the technical scope of the present invention.

The disclosure of Japanese Patent Application No. 2023-203197, filed on November 30, 2023, including the specification, drawings, and abstract, is incorporated herein by reference in its entirety.

### Industrial Applicability

The present invention can be applied to a culture apparatus and a culture system for culturing various cells.

### Reference Signs List

1 Culture system
2 Culture environment generation apparatus
20 Housing
201 Inner box
201a Culture chamber
201b Opening
202 Outer box
203 Insulation
204 Outer door
205 Inner door
206 Front plate
21 Heating
211, 212, 213, 214 Heater
22 Duct
22a Suction port
22b Discharge port
23 Circulation blower
24 Temperature sensor
26a, 26b Gas supply apparatus
27 Cover
28 Electric box
29 Control apparatus
31 Dehumidifying apparatus
32 Operation panel
4 Container unit
41 Supply container
411 Lower supply container
411a Container body
411b First port
411c Second port
412 Upper supply container
412a Container body
412b First port
412c Second port
42 Connection tube
43 Culture container
431 Container body
432 First port
433 Second port
434 Container-side sensor
44 Discharge container
441 Container body
442 First port
443 Second port
45 Supply tube
451 Pump attachment
46 Discharge tube
461 Pump attachment
5 Culture apparatus
51 Frame
511 Left frame
511a Front edge
511b Rear edge
511c Upper edge
511d Lower edge
511e Left handle
512 Right frame
512a Front edge
512b Rear edge
512c Upper edge
512d Lower edge
512e Right handle
513 Rear frame
514 Container disposition region
514a Supply container disposition region
514b Culture container disposition region
514c Discharge container disposition region
52 Rocker
521 Culture container support
521a Mount
521b Rocking support
522 Drive
53 Pump
531 Supply pump
532 Discharge pump
54 Supply container support
541 Lower support
542 Lower mount
543 Lower support member
543a Support element
543b Left side fixer
543c Right side fixer
544 Upper support
545 Upper mount
546 Upper support member
546a Support element
546b Left side fixer
546c Right side fixer
547 Gap
55 Terminal
56 Communicator
57 Controller
6 External terminal
61 Display
62 Communicator
63 Controller
P Packing
K Gas passage
M Machine room

## Claims

1. A culture apparatus disposed in a culture environment generation apparatus during cell culture, the culture apparatus comprising:
a frame that is open forward and includes a left frame and a right frame facing each other in a left-right direction; and
a rocker that is provided at the frame and rocks a culture container, wherein,
the frame has a container disposition region in which the culture container, a supply container, and a discharge container are disposed between the left frame and the right frame.

2. The culture apparatus according to Claim 1, wherein,
the culture container, the supply container, and the discharge container are disposed with their respective ports facing forward during cell culture.

3. The culture apparatus according to Claim 1, wherein,
the supply container, the culture container, and the discharge container are disposed in this order from above in the container disposition region during cell culture.

4. The culture apparatus according to Claim 1, wherein,
the frame includes a rear frame that connects the left frame and the right frame in the left-right direction and that supports the left frame and the right frame in a cantilevered manner.

5. The culture apparatus according to Claim 4, wherein,
the rocker is supported by the rear frame.

6. The culture apparatus according to Claim 5, wherein,
the rocker includes:
a culture container support on which the culture container is mounted; and
a drive that is supported by the rear frame and rocks the culture container support, and
the culture container support is supported by the rear frame in a cantilevered manner via the drive.

7. The culture apparatus according to Claim 1, wherein,
the left frame and the right frame each have a frame shape.

8. The culture apparatus according to Claim 1, wherein,
the frame is open upward, downward, and forward.

9. The culture apparatus according to Claim 1, wherein,
the left frame includes a left handle at a front end of the left frame, and
the right frame includes a right handle at a front end of the right frame.

10. The culture apparatus according to Claim 1, further comprising:
a pump that is provided at the frame and moves a culture medium between the supply container, the culture container, and the discharge container.

11. The culture apparatus according to Claim 10, wherein,
the pump includes:
a supply pump that supplies the culture medium contained in the supply container to the culture container; and
a discharge pump that discharges the culture medium contained in the culture container to the discharge container.

12. The culture apparatus according to Claim 11, wherein,
the supply pump and the discharge pump are provided on a front surface of the frame.

13. The culture apparatus according to Claim 12, wherein,
the supply pump is provided at an upper end on a front surface of one of the left frame and the right frame, and
the discharge pump is provided at a lower end on a front surface of another of the left frame and the right frame.

14. The culture apparatus according to Claim 12, wherein,
the supply container, the culture container, and the discharge container are connected to each other by at least one tube at a front side of the frame during cell culture, and
the tube is routed in an S-shape when viewed from a front side of the culture apparatus.

15. The culture apparatus according to Claim 1, further comprising:
a supply container support that is supported by the frame and on which the supply container is mounted during cell culture, in a region where the supply container is disposed within the container disposition region.

16. The culture apparatus according to Claim 15, wherein,
the supply container includes a lower supply container and an upper supply container connected by at least one tube,
the supply container support includes:
a lower mount on which the lower supply container is mounted; and
an upper mount on which the upper supply container is mounted, and
the lower mount and the upper mount overlap vertically and are offset in the left-right direction.

17. The culture apparatus according to Claim 16, wherein,
the lower mount and the upper mount are both inclined in a same direction.

18. The culture apparatus according to Claim 1, wherein,
the frame is configured to be capable of receiving the supply container, the culture container, and the discharge container in the container disposition region while the culture container, the supply container, and the discharge container are connected by at least one tube.

19. The culture apparatus according to Claim 1, further comprising:
a container unit that includes the culture container, the supply container, the discharge container, and at least one tube connecting the culture container, the supply container, and discharge container, wherein,
the tube is routed on a front side of the frame in an S-shape when viewed from a front side of the culture apparatus during cell culture.

20. A culture system, comprising:
the culture apparatus according to Claim 1; and
the culture environment generation apparatus that has a culture space accommodating the culture apparatus.
